# EUROPEAN PATENT APPLICATION

(11) **EP 3 181 082 A1**
(43) Date of publication of application: **21.06.2017**
(21) Application number: 16203956.4
(22) Date of filing: 14.12.2016
(51) Int. Cl.: A61B 18/14, A61B 18/00, A61B 90/00, A61B 5/021, A61M 25/00

(54) **TRANSLUMINAL ELECTRODE CATHETERS**

(30) Priority: 17.12.2015 US 201514972756
(71) Applicant: Pythagoras Medical Ltd., 56140 Herzliya (IL)
(72) Inventor: GROSS, Yossi, 73160 Moshav Mazor (IL); ZADOK, Yehuda, 5551021 Kiryat Ono (IL)
(74) Representative: White, Duncan Rohan

(57) **Abstract**

A first catheter (301) has a first plurality of longitudinally-distributed markers (304) thereon, one or more ablating electrodes (302) coupled to the first catheter, and all of the first plurality of markers located at least 20 cm along the catheter from a proximalmost one of the ablating electrodes. A second catheter (281) has a second plurality of longitudinally-distributed markers (284) thereon; and one or more stimulating electrodes (282) coupled to the second catheter, all of the second plurality of markers being disposed at least 20 cm from a proximalmost one of the stimulating electrodes. Each one of the second plurality of markers is at a respective distance from the proximalmost one of the stimulating electrodes that is equal to a distance (e.g., a predetermined, known distance) of a respective marker of the first plurality of markers from the proximalmost one of the ablating electrodes. Other embodiments are also described.

## Description

The present application claims priority from US patent application 14/972,756 to Gross et al., filed December 17, 2015, and entitled "Transluminal electrode catheters," which is incorporated herein by reference.

### FIELD OF THE INVENTION

Applications of the present invention relate generally to electrical manipulation of nerve tissue. Some applications of the present invention relate more specifically to apparatus and techniques for facilitating location, excitation, and/or ablation of nerve tissue.

### BACKGROUND

Hypertension is a prevalent condition in the general population, particularly in older individuals. Sympathetic nervous pathways, such as those involving the renal nerve, are known to play a role in regulating blood pressure. Ablation of renal nerve tissue from the renal artery is a known technique for treating hypertension.

### SUMMARY OF THE INVENTION

An expandable electrode unit is provided at the distal end of a catheter. For some applications the electrode unit comprises a flexible Printed Circuit Board (PCB) coupled to a scaffold, with one or more passthrough sites at which the PCB passes through the scaffold (or vice versa). Several configurations and optional features for the electrode unit are described. Typically the electrode unit is configured for performing targeted and controlled renal nerve ablation.

Techniques are described for avoiding inadvertently or unnecessarily ablating, during renal nerve ablation, nerve fibers that are not renal nerve fibers that are responsible for increasing blood pressure. For example, techniques are described for avoiding inadvertently ablating nerve fibers that carry motor or pain signals, and for avoiding ablating at more sites of the renal artery than is necessary.

A pair of catheter systems is described that facilitate ablation at a target site that has been previously (e.g., on a different day) identified.

Techniques are also described for performing targeted and controlled ablation at sites other than the renal artery.

There are therefore provided, in accordance with some applications of the invention, the following inventive concepts:
1. A method for use with a renal artery of a subject, the method including:
   (a) advancing a catheter to a first portion of a renal artery of the subject, the catheter having a plurality of electrodes coupled thereto;
   (b) using one or more of the electrodes to apply ablating energy at a first portion of the renal artery;
   (c) using one or more of the electrodes to apply excitatory current at a second portion of the renal artery that is at or efferent to the first portion;
   (d) using a physiological sensor, sensing a physiological response of the subject to the excitatory current and determining whether the physiological response is below a threshold response;
   (e) subsequently, based upon determining that the physiological response is below the threshold response, advancing the catheter distally;
   (f) subsequently, using one or more of the electrodes to apply excitatory current at a third portion of the renal artery that is the same as or efferent to the second portion;
   (g) using the physiological sensor, sensing a physiological response of the subject to the excitatory current applied at the third portion and determining whether the physiological response is above the threshold response; and
   (h) subsequently, based upon determining that the physiological response to the application of excitatory current at the third portion is above the threshold response, using one or more of the electrodes to apply ablating energy at the third portion.
2. The method according to inventive concept 1, further including repeating steps (d)-(h) at least twice.
3. The method according to inventive concept 1, wherein the second portion is efferent to the first portion, and the step of applying excitatory current at the second portion includes using the one or more electrodes to apply excitatory current at the second portion of the renal artery that is efferent to the first portion.
4. The method according to inventive concept 1, wherein the third portion is efferent to the second portion, and the step of applying ablating energy at the third portion includes using the one or more electrodes to apply ablating energy at the third portion of the renal artery that is efferent to the second portion.
5. The method according to inventive concept 1, further including, prior to the step of applying ablating energy at the first portion:
   using one or more of the electrodes to apply excitatory current at the first portion; and
   using the physiological sensor, sensing a physiological response of the subject to the excitatory current applied at the first portion and determining whether the physiological response to the excitatory current applied at the first portion is above a threshold response, wherein the step of applying ablating energy at the first portion is performed based upon determining that the physiological response to the excitatory current applied at the first portion is above the threshold response.
6. The method according to inventive concept 1, wherein:
   an expandable electrode device that includes the plurality of electrodes is coupled to a distal portion of the catheter, and
   the method further includes:
      placing the one or more of the electrodes in contact with the renal artery by expanding the electrode device prior to the step of using the one or more of the electrodes to apply ablating energy at the first portion of the renal artery;
      between the steps of (i) using the one or more of the electrodes to apply ablating energy at the first portion of the renal artery, and (ii) using the one or more of the electrodes to apply excitatory current at the second portion of the renal artery, placing the one or more of the electrodes in contact with the renal artery by re-expanding the electrode device.
7. The method according to inventive concept 1, further including, subsequently to the advancing and prior to the applying ablating energy at the third portion, (i) using one or more of the electrodes to apply excitatory current at the third portion, and (ii) and using the physiological sensor, sensing that a physiological response of the subject to the excitatory current applied at the third portion is above a threshold response, and wherein the applying of ablating energy at the third portion includes applying ablating energy at the third portion in response to the sensing that the physiological response of the subject to the excitatory current applied at the third portion is above a threshold response.
8. The method according to inventive concept 1, wherein the physiological sensor is a blood pressure sensor, and sensing that the physiological response is below the threshold response includes sensing that a blood pressure increase is less than a threshold blood pressure increase.
9. The method according to inventive concept 1, wherein the physiological sensor is a blood pressure sensor, and sensing that the physiological response is below the threshold response includes sensing that the physiological response is below the threshold response using the blood pressure sensor.
10. The method according to any one of inventive concepts 1-9, further including repeating steps (d)-(h) until at least one of the electrodes of the plurality of electrodes is disposed less than a distance of 0.5 cm of a kidney supplied with blood by the renal artery.
11. The method according to inventive concept 10, further including:
   subsequently to the at least one of the electrodes becoming disposed within the distance of the kidney, beginning to move the catheter proximally and continuing to move the catheter proximally until the catheter is withdrawn from the subject; and
   after beginning to move the catheter proximally and until the catheter is withdrawn from the subject, not applying ablating energy to the renal artery.
12. The method according to any one of inventive concepts 1-9, wherein:
   using one or more of the electrodes to apply ablating energy at the first portion includes using one or more of the electrodes to apply an application of ablating energy at the first portion;
   using one or more of the electrodes to apply excitatory current at the second portion includes using one or more of the electrodes to apply a second application of excitatory current at the second portion; and
   the method further includes, prior to applying the second application of excitatory current:
      using one or more of the electrodes to apply a first application of excitatory current at the second portion;
      using the physiological sensor, sensing a physiological response of the subject to the first application of excitatory current and determining whether the physiological response to the first application of excitatory current is above the threshold response;
      and
      subsequently, based upon determining that the physiological response to the first application of excitatory current is above the threshold response, using one or more of the electrodes to apply another application of ablating energy to the renal artery.
13. The method according to inventive concept 12, wherein using one or more of the electrodes to apply the other application of ablating energy includes using one or more of the electrodes to apply the other application of ablating energy at the second portion.
14. A method for treating a subject, the method including:
   advancing a catheter to a first portion of a renal artery of the subject, the catheter having a plurality of electrodes coupled thereto;
   using one or more of the electrodes, during a first stimulation, exciting a renal nerve of the subject;
   using a physiological sensor, sensing a physiological response of the subject to the first stimulation;
   in response to the sensing, using one or more of the electrodes to pass a first ablating current through the renal nerve;
   advancing the catheter to a second portion of the renal artery, the second portion being distal to the first portion;
   using one or more of the electrodes, during a second stimulation, exciting the renal nerve;
   using the physiological sensor, sensing a physiological response of the subject to the second stimulation; and
   in response to the sensing, using one or more of the electrodes to pass a second ablating current through the renal nerve.
15. The method according to inventive concept 14, wherein exciting the renal nerve during the first stimulation includes exciting the renal nerve by passing an excitatory electric current between two of the electrodes.
16. Apparatus, including:
   a catheter, having a proximal portion, and a distal portion configured to be advanced into a subject, the catheter providing electrical communication between the proximal portion and the distal portion; and
   an electrode device, coupled to the distal portion of the catheter, having a compressed state and an expanded state, and including one or more subunits, each subunit including a scaffold and a printed circuit board (PCB), and defining at least one passthrough site at which one element selected from the group consisting of: the scaffold and the PCB passes through the other element of the group.
17. The apparatus according to inventive concept 16, wherein each subunit includes an electrode, and the PCB defines a conductor that electrically connects the electrode to the catheter.
18. The apparatus according to inventive concept 16, wherein the one selected element is the PCB and the other selected element is the scaffold.
19. The apparatus according to inventive concept 16, wherein:
   the scaffold is shaped to define an opening through the scaffold at the at least one passthrough site,
   at the at least one passthrough site, the PCB passes through the scaffold via the opening, and
   the opening increases flexibility of the scaffold adjacent the opening, and thereby facilitates transition of the electrode device between the compressed state and the expanded state.
20. The apparatus according to inventive concept 16, wherein each subunit has at least one hinge region that facilitates transition of the electrode device between the compressed state and the expanded state, and wherein the at least one passthrough site is disposed at the at least one hinge region.
21. The apparatus according to any one of inventive concepts 16-20, wherein:
   the electrode device has a longitudinal axis that is collinear with the distal portion of the catheter,
   the one or more subunits are a plurality of subunits, arranged circumferentially around the longitudinal axis, and
   the electrode device is transitionable between the compressed state and the expanded state by changing a distance, from the longitudinal axis, of at least a portion of each subunit of the plurality of subunits.
22. The apparatus according to inventive concept 21, wherein the one or more subunits are elongate and are aligned with the longitudinal axis.
23. The apparatus according to any one of inventive concepts 16-20, wherein the at least one passthrough site includes a first passthrough site and a second passthrough site.
24. The apparatus according to inventive concept 23, wherein the first passthrough site is disposed at a first hinge region of the subunit, and the second passthrough site is disposed at a second hinge region of the subunit.
25. The apparatus according to inventive concept 23, wherein the first passthrough site and the second passthrough site substantially define, therebetween, a hinge region of the subunit.
26. The apparatus according to inventive concept 25, wherein:
   the hinge region includes a first hinge region of the subunit,
   each subunit further has a second hinge region, defines an electrode region between the first hinge region and the second hinge region, and includes at least one electrode within the electrode region.
27. The apparatus according to inventive concept 23, wherein each subunit defines an electrode region between the first passthrough site and the second passthrough site, and includes at least one electrode within the electrode region.
28. The apparatus according to inventive concept 27, wherein:
   each subunit is elongate, and has a proximal region proximal to the first passthrough site, and a distal region distal to the second passthrough site, and
   at the proximal region and the distal region, the PCB is disposed on a radially-outward-facing side of the scaffold.
29. The apparatus according to inventive concept 27, wherein at the electrode region the PCB is disposed on a radially-outward-facing side of the scaffold.
30. The apparatus according to inventive concept 29, wherein:
   each subunit is elongate, and has a proximal region proximal to the first passthrough site, and a distal region distal to the second passthrough site, and
   at the proximal region and the distal region, the PCB is disposed on a radially-inward-facing side of the scaffold.
31. The apparatus according to inventive concept 29, wherein:
   each subunit is elongate, and has a proximal region proximal to the first passthrough site, and a distal region distal to the second passthrough site, and
   at the proximal region and the distal region, the PCB is disposed on a radially-outward-facing side of the scaffold.
32. The apparatus according to inventive concept 31, wherein:
   each subunit defines (i) a third passthrough site between the first passthrough site and the electrode region, and (ii) a fourth passthrough site between the second passthrough site and the electrode region, and
   between the first and third passthrough sites, and between the second and fourth passthrough sites, the PCB is disposed on a radially-inward-facing side of the scaffold.
33. The apparatus according to inventive concept 27, wherein at the electrode region the PCB is disposed on a radially-inward-facing side of the scaffold.
34. The apparatus according to inventive concept 33, wherein:
   each subunit is elongate, and has a proximal region proximal to the first passthrough site, and a distal region distal to the second passthrough site, and
   at the proximal region and the distal region, the PCB is disposed on a radially-outward-facing side of the scaffold.
35. The apparatus according to inventive concept 27, wherein each subunit is elongate, and has a proximal end, a distal end, and a length therebetween.
36. The apparatus according to inventive concept 35, wherein a straight line drawn between the first and second ends is generally parallel with the longitudinal axis.
37. The apparatus according to inventive concept 27, wherein each subunit defines a third passthrough site and a fourth passthrough site, and the electrode region is also between the third passthrough site and the fourth passthrough site.
38. The apparatus according to inventive concept 27, wherein the electrode region is generally straight and parallel with the longitudinal axis.
39. The apparatus according to inventive concept 27, wherein the electrode region is convex.
40. The apparatus according to inventive concept 27, wherein each subunit includes a plurality of electrodes within the electrode region.
41. The apparatus according to inventive concept 40, wherein the electrodes of the plurality of electrodes are disposed equidistant from the longitudinal axis.
42. The apparatus according to inventive concept 23, wherein the scaffold has a hinge adjacent at least one of the passthrough sites.
43. The apparatus according to inventive concept 42, wherein the hinge includes a spring.
44. Apparatus, including:
   a catheter, having a proximal portion, and a distal portion configured to be advanced into a subject, the catheter providing electrical communication between the proximal portion and the distal portion; and
   an electrode device, coupled to the distal portion of the catheter, having a compressed state and an expanded state, and including one or more subunits, each subunit:
      having at least one hinge region that facilitates transition of the electrode device between the compressed state and the expanded state, and
      including an electrode, a scaffold, and a conductor that electrically connects the catheter to the electrode, the conductor coupled to the scaffold such that transition of the electrode device between the compressed state and the expanded state alters a size of a gap, within each hinge region, between the conductor and the scaffold.
45. Apparatus, including:
   a catheter, having a proximal portion, and a distal portion configured to be advanced into a subject, the catheter providing electrical communication between the proximal portion and the distal portion; and
   an electrode device, coupled to the distal portion of the catheter, having a compressed state and an expanded state, and including one or more subunits, each subunit:
      having at least one hinge region that facilitates transition of the electrode device between the compressed state and the expanded state, and
      including an electrode, a scaffold, and a conductor that electrically connects the catheter to the electrode, the conductor coupled to the scaffold such that transition of the electrode device from the compressed state toward the expanded state causes sliding between the conductor and the scaffold.
46. Apparatus for use with a first catheter having a first plurality of markers thereon, one or more ablating electrodes being coupled to the first catheter, all of the first plurality of markers being located at least 20 cm along the catheter from a proximalmost one of the ablating electrodes, the apparatus including:
   a second catheter having a second plurality of markers thereon; and
   one or more stimulating electrodes coupled to the second catheter,
      all of the second plurality of markers being disposed at least 20 cm from a proximalmost one of the stimulating electrodes, and
      each one of the second plurality of markers being at a respective distance from the proximalmost one of the stimulating electrodes that is equal to a distance of a respective marker of the first plurality of markers from the proximalmost one of the ablating electrodes.
47. The apparatus according to inventive concept 46, further including the first catheter.
48. The apparatus according to inventive concept 46, wherein:
   the first catheter includes a first handle, and a first flexible and transluminally-advanceable shaft, and, the first plurality of markers are disposed on the first shaft, and
   the first catheter includes a second handle, and a second flexible and transluminally-advanceable shaft, and, the second plurality of markers are disposed on the second shaft.
49. The apparatus according to any one of inventive concepts 46-48, wherein the second catheter includes an extracorporeal control unit that is electrically coupled to the one or more stimulating electrodes, and is configured to drive the one or more stimulating electrodes to apply an excitatory current having a frequency of 5-50 Hz.
50. A method including:
   using one or more of a plurality of stimulating electrodes, exciting tissue of a renal nerve of a subject by passing an excitatory electric current through the tissue;
   detecting a musculoskeletal motor response of the subject to the excitation;
   in response to detecting the motor response, deciding whether to ablate the tissue; and
   in response to deciding to ablate the tissue, ablating the tissue by passing an ablating signal through the tissue.
51. The method according to inventive concept 50, wherein the excitatory electric current has a frequency of 1-250 Hz.
52. The method according to inventive concept 50, wherein detecting the motor response of the subject includes detecting movement of a leg of the subject.
53. The method according to inventive concept 50, wherein detecting the motor response of the subject includes detecting movement of a hand of the subject.
54. The method according to inventive concept 50, wherein detecting the motor response of the subject includes detecting movement of a lower abdominal muscle of the subject.
55. The method according to inventive concept 50, wherein passing the ablating signal through the tissue includes using one or more ablating electrodes to pass an ablating electric current through the tissue.
56. The method according to inventive concept 50,
   wherein exciting the tissue includes exciting the tissue at a plurality of sites, and
   wherein deciding whether to ablate the tissue includes deciding to ablate the tissue at a first one of the sites, and not at a second one of the sites, in response to detecting a greater motor response of the subject to the excitation at the second one of the sites, relative to the first one of the sites.
57. The method according to any one of inventive concepts 50-56,
   wherein the excitatory electric current is a first excitatory electric current,
   wherein the method further includes:
   in response to detecting the motor response to the first excitatory electric current, using one or more of the plurality of stimulating electrodes to pass a second excitatory electric current through the tissue, the second excitatory electric current having a frequency of 1-150 Hz; and
   detecting a musculoskeletal motor response of the subject to the second excitatory electric current, and
   wherein deciding whether to ablate the tissue includes deciding to not ablate the tissue, in response to detecting the motor response to the second excitatory electric current.
58. The method according to inventive concept 57, wherein the second excitatory electric current has a frequency of 90-110 Hz, the method including using one or more of the plurality of stimulating electrodes to pass the second excitatory electric current through the tissue.
59. The method according to any one of inventive concepts 50-56, wherein the method further includes setting a power of the ablating signal in response to detecting the motor response.
60. The method according to inventive concept 59, wherein detecting the motor response includes detecting a magnitude of the motor response, and wherein setting the power of the ablating signal includes setting the power of the ablating signal in response to the magnitude of the motor response.
61. A method including:
   using one or more of a plurality of stimulating electrodes, exciting tissue of a renal nerve of a subject by passing an excitatory electric current through the tissue;
   determining a level of pain experienced by the subject in response to the electric current;
   in response to the level of pain, deciding whether to ablate the tissue; and
   in response to deciding to ablate the tissue, using one or more of the plurality of electrodes to ablate the tissue by passing an ablating electric current through the tissue.
62. A method for treating a subject, the method including:
   using one or more stimulating electrodes disposed within a renal artery of the subject, passing one or more excitatory electric currents through tissue of a subject;
   determining respective levels of pain experienced by the subject in response to the electric currents;
   in response to the levels of pain, determining an approximate location of a renal nerve of the subject; and
   using one or more ablating electrodes disposed within the renal artery, passing an ablating current through the renal nerve.
63. The method according to inventive concept 62, further including setting a power of the ablating current in response to the levels of pain.
64. Apparatus including:
   a catheter; and
   an expandable structure disposed at a distal end of the catheter, the expandable structure including:
      a plurality of elongate elements, the elongate elements being (a) generally parallel to a central longitudinal axis of the catheter, (b) expandable radially outward, and (c) collapsible radially inward,
      each one of the elongate elements including:
         a printed circuit board (PCB); and
         one or more electrodes connected to the PCB and at least partially surrounding a respective portion of the PCB.
65. The apparatus according to inventive concept 64, wherein a length of the expandable structure is 15-25 mm.
66. The apparatus according to any one of inventive concepts 64-65, wherein an outer diameter of the expandable structure is 1-2.5 mm, when the expandable structure is fully compressed.
67. The apparatus according to inventive concept 66, wherein an outer diameter of the expandable structure is 4-8 mm, when the expandable structure is fully expanded.
68. The apparatus according to inventive concept 67, wherein, when the expandable structure is not subject to any radial force, an outer diameter of the expandable structure (i) is 2-4 mm, and (ii) is larger than the outer diameter of the expandable structure when the expandable structure is fully collapsed.
69. The apparatus according to any one of inventive concepts 64-65, wherein each PCB includes one or more metal pads on a surface of the PCB, the electrodes being connected to the PCB by being connected to respective ones of the metal pads.
70. The apparatus according to inventive concept 69, wherein at least one of the metal pads on each PCB is not connected to any of the electrodes.
71. The apparatus according to any one of inventive concepts 64-65, wherein each one of the electrodes is shaped to define a ring surrounding a body of one of the elongate elements, the ring including:
   an electrically-conductive outward-facing partial-ring portion; and
   an electrically-insulating inward-facing partial-ring portion.
72. The apparatus according to inventive concept 71, wherein the outward-facing partial-ring portion has an area of 0.5-3 mm^2.
73. A method of assembling a transluminal electrode catheter, including:
   at a distal end of a transluminally-advanceable catheter, placing a metal pad of an elongate printed circuit board (PCB) in electrical contact with a wire that extends proximally along the catheter, by electrically connecting the PCB to the wire; and
   threading the PCB through a platinum-iridium ring; and
   securing the ring to the PCB such that the ring is in electrical contact with the metal pad.
74. The method according to inventive concept 73, wherein securing the ring includes crimping the ring to the PCB.
75. The method according to inventive concept 73, wherein securing the ring includes soldering the ring to the PCB.
76. The method according to inventive concept 73, wherein securing the ring includes welding the ring to the PCB.
77. A method for locating and ablating a nerve that regulates activity of a duodenum of a subject, the method including:
   using one or more stimulating electrodes disposed within a gastrointestinal (GI) tract of the subject, passing one or more excitatory electric currents through a wall of the GI tract;
   detecting activity of the duodenum that is caused by the electric currents;
   in response to detecting the activity of the duodenum, locating the nerve; and
   in response to locating the nerve, ablating the nerve.
78. The method according to inventive concept 77, wherein detecting activity of the duodenum includes using imaging to detect activity of the duodenum.
79. The method according to inventive concept 77, further including, after ablating the nerve, using the one or more stimulating electrodes to pass one or more excitatory electric currents through the wall of the GI tract.
80. The method according to any one of inventive concepts 77-79, further including, prior to passing the one or more excitatory electric currents through the wall of the GI tract, using one or more sensing electrodes disposed within the GI tract of the subject to detect activity of the duodenum, wherein passing the one or more excitatory electric currents through the wall of the GI tract includes passing the one or more excitatory electric currents through the wall of the GI tract in response to the activity detected using the sensing electrodes.
81. A method for locating and ablating a nerve that regulates activity of a duodenum of a subject, the method including:
   using one or more electromyographic electrodes disposed within a gastrointestinal (GI) tract of the subject, detecting activity of the duodenum;
   in response to detecting the activity of the duodenum, locating the nerve; and
   in response to locating the nerve, ablating the nerve.
82. A method for locating and ablating a nerve that regulates activity of a duodenum of a subject, the method including:
   advancing, into the duodenum of a subject, a distal portion of a catheter that includes an electrode device;
   using the catheter to locate the nerve to a first degree of accuracy; and
   subsequently, locating the nerve to a second degree of accuracy that is greater than the first degree of accuracy, by (i) using the electrode device to apply an excitatory current to the duodenum, and (ii) using the catheter to detect a change in activity of the duodenum in response to the excitatory current.
83. The method according to inventive concept 82, wherein the catheter includes at least one EMG electrode, and using the catheter to locate the nerve to the first degree of accuracy includes using the EMG electrode to locate the nerve to the first degree of accuracy.
84. The method according to inventive concept 82, wherein the catheter includes at least one EMG electrode, and using the catheter to locate the nerve to the second degree of accuracy includes using the EMG electrode to detect the change in activity of the duodenum.
85. The method according to any one of inventive concepts 82-84, wherein the catheter includes an imaging device, and using the catheter to locate the nerve to the first degree of accuracy includes using the imaging device to locate the nerve to the first degree of accuracy.
86. The method according to inventive concept 85, wherein the imaging device is a camera, and using the imaging device to locate the nerve to the first degree of accuracy includes using the camera to locate the nerve to the first degree of accuracy.
87. The method according to inventive concept 85, wherein the imaging device is an ultrasound transceiver, and using the imaging device to locate the nerve to the first degree of accuracy includes using the ultrasound transceiver to locate the nerve to the first degree of accuracy.
88. The method according to any one of inventive concepts 82-84, wherein the catheter includes an imaging device, and using the catheter to locate the nerve to the second degree of accuracy includes using the imaging device to detect the change in activity of the duodenum.
89. The method according to inventive concept 88, wherein the imaging device is a camera, and using the imaging device to detect the change in activity of the duodenum includes using the camera to detect the change in activity of the duodenum.
90. The method according to inventive concept 88, wherein the imaging device is an ultrasound transceiver, and using the imaging device to detect the change in activity of the duodenum includes using the ultrasound transceiver to detect the change in activity of the duodenum.
91. Apparatus, comprising:
   a first catheter, having a first plurality of longitudinally-distributed markers thereon;
   one or more ablating electrodes coupled to the first catheter;
   a second catheter having a second plurality of longitudinally-distributed markers thereon; and
   one or more stimulating electrodes coupled to the second catheter,
   wherein:
   all of the first plurality of markers are located at least 20 cm along the first catheter from a proximalmost one of the ablating electrodes,
   all of the second plurality of markers are disposed at least 20 cm along the second catheter from a proximalmost one of the stimulating electrodes, and
   a distance along the second catheter between each one of the second plurality of markers and the proximalmost one of the stimulating electrodes, is equal to a distance along the first catheter between a respective marker of the first plurality of markers and the proximalmost one of the ablating electrodes.
92. The apparatus according to inventive concept 91, further comprising an extracorporeal control unit that is electrically coupled to the one or more ablating electrodes, and is configured to drive the one or more ablating electrodes to apply a high-frequency ablation signal.
93. The apparatus according to inventive concept 91, further comprising an extracorporeal control unit that is electrically coupled to the one or more stimulating electrodes, and is configured to drive the one or more stimulating electrodes to apply an excitatory current having a frequency of 5-50 Hz.

The present invention will be more fully understood from the following detailed description of applications thereof, taken together with the drawings, in which:

### BRIEF DESCRIPTION OF THE DRAWINGS

Figs. 1A-B are schematic illustrations of a system comprising an electrode device and a catheter, in accordance with some applications of the invention;
Fig. 2 is a schematic illustration of an electrode device, in accordance with some applications of the invention;
Figs. 3A-B are schematic illustrations of a technique for assembling the system, in accordance with some applications of the invention;
Fig. 4 is a schematic illustration of an electrode device, in accordance with some applications of the invention;
Fig. 5 is a schematic illustration of an electrode device, in accordance with some applications of the invention;
Figs. 6 and 7 are flow charts of at least some steps in respective techniques for use with a renal artery of a subject, in accordance with some applications of the invention;
Figs. 8A-H are schematic illustrations of a technique for performing renal nerve ablation, in accordance with some applications of the invention;
Figs. 9A-B are schematic illustrations of a pair of catheter systems comprising a first catheter system and a second catheter system, in accordance with some applications of the invention; and
Figs. 10-11 are, respectively, a schematic illustration and a flow chart showing at least some steps of a technique for facilitating nerve ablation at a site other than the renal artery, in accordance with some applications of the invention.

### DETAILED DESCRIPTION OF EMBODIMENTS

The apparatus and methods described herein are typically for use with a renal artery of a subject, in particular for renal nerve ablation (also called renal denervation) and facilitating decisions related thereto. However, it is to be noted that applications of the invention can also be used with other anatomical sites, in particular those described herein.

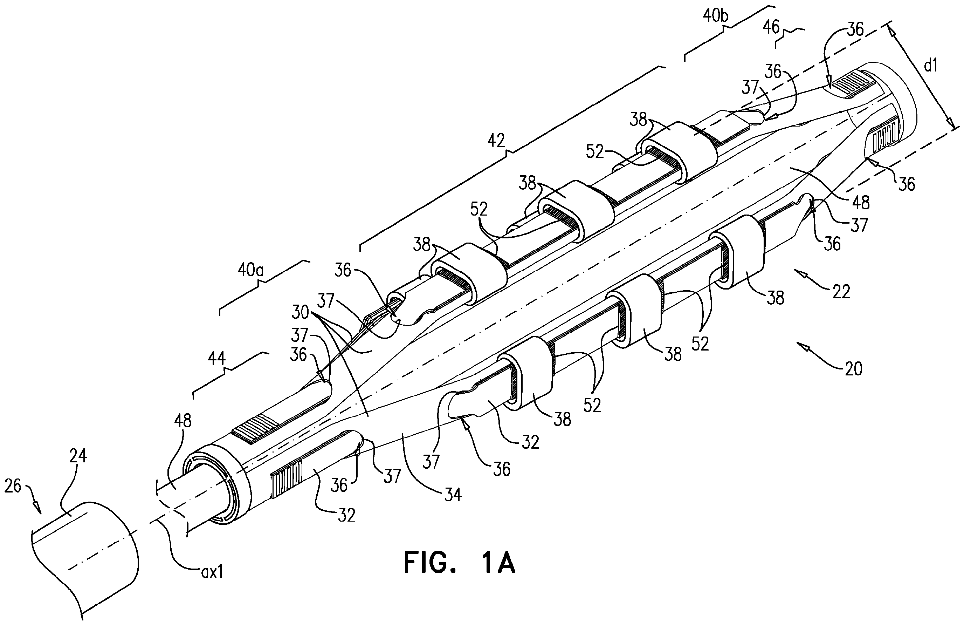

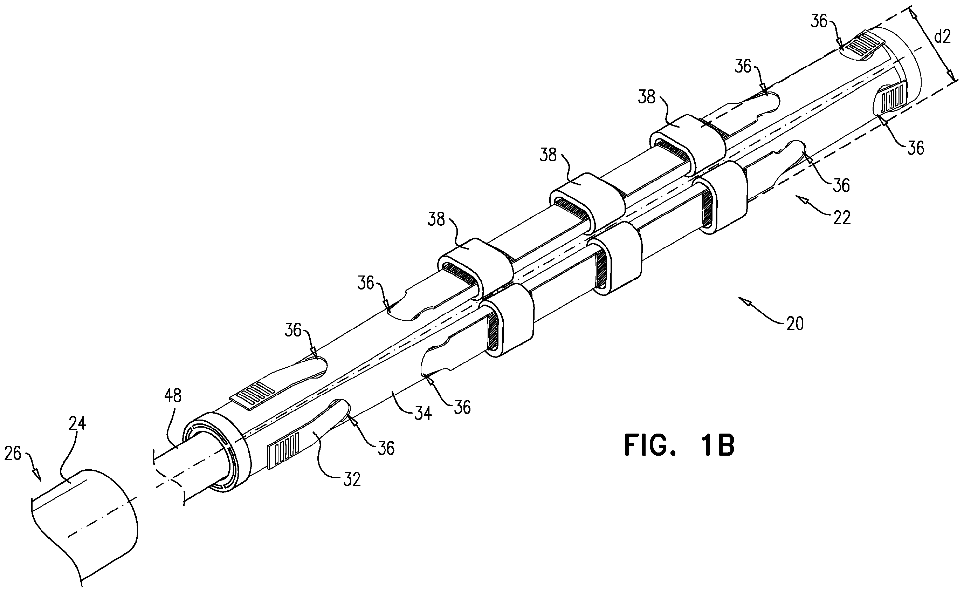

Reference is made to Figs. 1A-B, which are schematic illustrations of a system 20 comprising an electrode device 22 and a catheter 24, in accordance with some applications of the invention. Catheter 24 has a proximal portion (not shown), and a distal portion 26 configured to be advanced into a subject (e.g., transluminally). Catheter 24 provides electrical communication between the proximal portion (e.g., a control unit 240, described hereinbelow) and distal portion 26, e.g., by comprising a plurality of wires that extend along the catheter. Electrode device 22 is shown separately from catheter 24, but in device 22 the electrode device is coupled to distal portion 26 of the catheter. Electrode device 22 is an expandable structure. Fig. 1A shows electrode device 22 in an expanded state thereof, and Fig. 1B shows the electrode device in a compressed state thereof. Typically, electrode device 22 is used to apply current to tissue when in its expanded state, and is moved within the vasculature of the subject when in its compressed state.

Electrode device 22 comprises one or more subunits 30 (typically a plurality of subunits, such a 2-6, e.g., 4 subunits). Each subunit 30 comprises a scaffold 34 and a Printed Circuit Board (PCB) 34, the substrate of the PCB typically being flexible. Typically, electrode device 22 has a longitudinal axis ax1 that is collinear with distal portion 26 of catheter 24. Typically, each scaffold 34 and PCB 32 is elongate, and each PCB is coupled to its respective scaffold in an alignment such that each subunit is elongate. Subunits 30 are typically aligned with, and arranged circumferentially around, longitudinal axis ax1, and the subunits are arranged circumferentially around the longitudinal axis. Although at least in the expanded state subunits 30 are not straight, their alignment with axis ax1 may be described as "parallel" insomuch as, for each subunit, a straight line drawn between its ends is generally parallel with the axis ax1.

Each subunit 30 defines at least one passthrough site 36 (typically at least two, e.g., four passthrough sites) at which PCB 32 passes through scaffold 34, or vice versa. Typically, and as shown in Figs. 1A-B, at each passthrough site scaffold 34 defines an opening 37 through the scaffold, through which PCB 32 passes.

Each subunit 30 defines an electrode region 42 and comprises at least one electrode 38 within the electrode region. Each subunit 30 defines at least one hinge region 40, e.g., a first hinge region 40a and a second hinge region 40b, and electrode region 42 is disposed between the hinge regions. For some applications each opening 37 increases the flexibility of scaffold 34 adjacent the opening, and thereby facilitates transition of the electrode device between the compressed state and the expanded state. For some applications, and as shown, at least one passthrough site 36 is disposed at each hinge region. For example, in the embodiment shown, hinge region 40a is substantially defined by and between (e.g., is delimited by) a pair of passthrough sites 36, and hinge region 40b is substantially defined by and between another pair of passthrough sites.

Typically, at electrode region 42, PCB 32 is disposed on a radially-outward-facing side of scaffold 34. Typically, PCB 32 is disposed on the radially-outward-facing side of scaffold 34 also at (i) a proximal region 44 of device 22 (e.g., a region proximal to the proximal-most passthrough site 36, and/or proximal to hinge region 40a) and (ii) a distal region 46 of the device (e.g., a region distal to the distal-most passthrough site 36, and/or distal to hinge region 40b). That is, in a proximal-to-distal direction along electrode device 22, PCB 32 (i) begins on the radially-outward-facing side of scaffold 34 at proximal region 44, (ii) passes through the scaffold to be disposed on a radially-inward-facing side of the scaffold at hinge region 40a, (iii) passes back through the scaffold to be disposed on the radially-outward-facing side of the scaffold at electrode region 42, (iv) passes through the scaffold to be disposed on a radially-inward-facing side of the scaffold at hinge region 40b, and (v) passes back through the scaffold to be disposed on the radially-outward-facing side of the scaffold at distal region 46.

For some applications, PCB 32 is alternatively disposed on the radially-inward-facing side of scaffold 34 at proximal region 44 and distal region 46.

For some applications, PCB 32 is not directly attached (e.g., adhered) to scaffold 34 in one or more regions of electrode device 22. For example, PCB 32 may not be directly attached to scaffold 34 in hinge regions 40. It is hypothesized that for some applications this facilitates articulation of device 22 at hinge regions 40.

Expansion and compression of device 22 comprises changing a distance, from axis ax1, of at least a portion of each subunit. Typically, that portion is electrode region 42. For some applications, and as shown, electrode region 42 is generally straight and/or flat, and in both the compressed and expanded states each electrode 38 of a particular subunit 30 is the same distance away from axis ax1 as are the other electrodes of that subunit. That is, during expansion of device 22, the rates at which all of the electrodes 38 of a particular subunit 30 move away from axis ax1, are equal.

Typically, the expansion and contraction of device 22 is effected by moving a control rod 48 with respect to catheter 24. For example, and as shown, control rod 48 may be attached to distal region 46 of device 22, while catheter 24 is attached to proximal region 44. Sliding of control rod 48 distally with respect to catheter 24 elongates the device such that it transitions into its contracted state, and sliding of the control rod proximally with respect to the catheter shortens the device such that it transitions into its expanded state. The above-described arrangement of electrode device 22, in particular passthrough sites 36 (e.g., openings 37), facilitates its controlled expansion and contraction, such that all electrodes 38 can be reliably placed in contact with surrounding tissue.

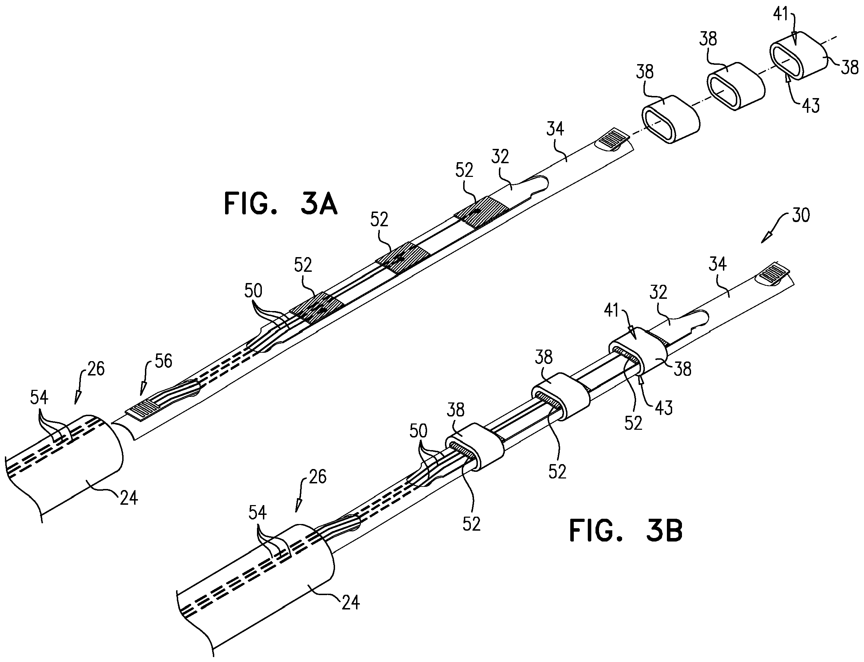

Each PCB 32 defines at least one conductor 50 (not shown in Figs. 1A-B, but shown in Figs. 3A-B) that electrically connects a respective electrode 38 to catheter 24. Typically, each PCB 32 defines at least one conductive pad 52 that facilitates this electrical connection, e.g., as described with reference to Figs. 3A-B. For applications in which each subunit comprises a plurality of electrodes 38 (e.g., three electrodes as shown), each PCB typically defines a corresponding plurality of conductors 50, and a corresponding plurality of conductive pads 52.

For some applications, electrodes 38 are coated with a coating of iridium oxide and/or titanium nitrate, so as to increase a charge-transfer interface of the electrode relative to if the electrode were not coated with the coating.

Each subunit 30 may comprise 1-3 electrodes (e.g., one electrode, two electrodes, or three electrodes). For some applications, a distance along the subunit between its end electrodes (i.e., distal-most electrode and the proximal-most electrode) is 2-10 mm (e.g., 4-8 mm, such as 6 mm).

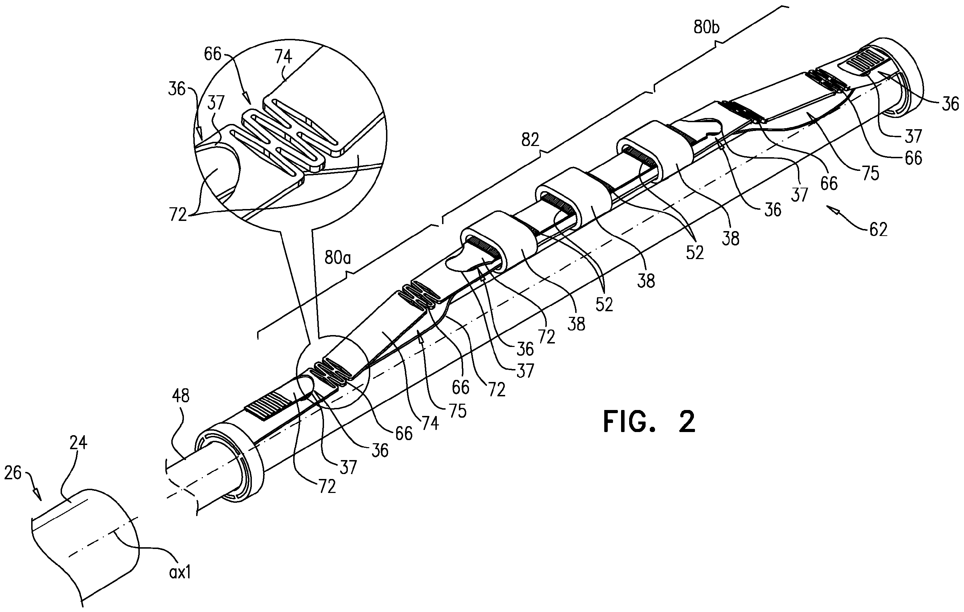

Reference is now made to Fig. 2, which is a schematic illustration of an electrode device 62, in accordance with some applications of the invention. Fig. 2 shows only one subunit 70 of device 62, but it is to be understood that device 62 typically comprises a plurality of subunits, e.g., as does device 22, *mutatis mutandis.*

System 20 may comprise electrode device 62 instead of electrode device 22, *mutatis mutandis.* Electrode device 62 is typically identical to electrode device 22, described hereinabove, except where noted. Each subunit 70 of device 62 comprises a PCB 72 that is typically identical to PCB 32, *mutatis mutandis;* and a scaffold 74 that is typically identical to scaffold 34, *mutatis mutandis,* except for the presence of hinges 66 position of passthrough sites 36 and openings 37.

Each subunit 70 defines an electrode region 82 and comprises at least one electrode 38 within the electrode region. Each subunit 70 defines at least one hinge region 80, e.g., a first hinge region 80 and a second hinge region 80b, and electrode region 82 is disposed between the hinge regions. A hinge 66 is disposed adjacent to at least one (and typically all) of passthrough sites 36. Typically, each hinge region 80 is substantially defined by and between (e.g., is delimited by) a pair of passthrough sites 36, and includes a pair of hinges 66, which are disposed closer together than are the pair of passthrough sites. Hinges 66 are hypothesized to further facilitate transition of the electrode device between the compressed state and the expanded state, and for some applications, to further facilitate the transition such that during expansion of the electrode device the rates at which all of the electrodes 38 of a particular subunit 70 move away from axis ax1, are equal.

Hinge 66 may comprise a distinct hinge element that couples adjacent portions of scaffold 74, or may be defined by a more flexible part of the scaffold. For example, and as shown, hinge 66 may be formed by removing material from (e.g., cutting) scaffold 74, e.g., to form a spring.

For some applications, a gap 75 exists between the PCB (e.g., conductors 50 thereof) and the scaffold of the electrode device (e.g., device 22 or 62), typically within the hinge region(s) of the device. Gap 75 is labeled in Fig. 2. For some such applications, transitioning of the electrode device between its compressed and expanded states alters a size of gap 75.

For some applications, the electrode device is configured such that transitioning of the electrode device between its compressed and expanded states causes sliding between the PCB (e.g., conductors 50 thereof) and the scaffold of the device, e.g., at passthrough sites 36 (e.g., openings 37).

Reference is now made to Figs. 3A-B, which are schematic illustrations of a technique for assembling system 20, in accordance with some applications of the invention. Fig. 3A shows catheter 24 and electrodes 38 separate from PCB 32, e.g., before assembly. Fig. 3B shows catheter 24 and electrodes 38 connected to PCB 32, e.g., after assembly. For clarity, only one subunit 30 is shown, although it is to be understood that the technique described may be used to assemble multiple subunits.

For some applications, and as shown, each electrode 38 is a ring (that may be elliptical (e.g., circular), or another shape such as a square or rectangle) made of a metal, e.g., a platinum-iridium alloy. PCB 32 is threaded through electrode 38; each electrode therefore surrounds a respective portion of the PCB. The electrode is secured to the PCB such that the electrode (i.e., the ring) is in electrical contact with a respective conductive pad 52. For example, the electrode may be crimped (i.e., deformed into a flatter shape), soldered, or laser welded to the PCB (e.g., to pad 52). Alternatively, electrode may be a C-shape, and may therefore only partly surround the respective portion of the PCB.

For some applications, the ring electrodes have an electrically-conductive outward-facing partial-ring portion 41 (i.e., the portion that faces radially outward from the device), and an electrically-insulating inward-facing partial-ring portion 43 (i.e., the portion that faces radially inward from the device), such that current is applied only by the portion of the ring that faces radially outward from axis ax1. For some applications, the outward-facing partial-ring portion of each electrode has an area of 0.5-3 mm^2.

At a distal end of catheter 24, pads 52 are placed in electrical contact with wires 54 that extend proximally along the catheter by electrically connecting PCB 32 to the catheter. For example, PCB 32 may define an electrical interface 56 (e.g., comprising soldering or welding pads) to facilitate electrically connecting wires 54 to conductors 50, e.g., by soldering or welding.

When electrodes 38 are rings, the electrodes are typically in contact with the scaffold. Therefore when electrodes 38 are rings, the scaffold typically comprises an insulating material (e.g., primarily or in a coating), such that the scaffold does not provide a conductive path between the electrodes.

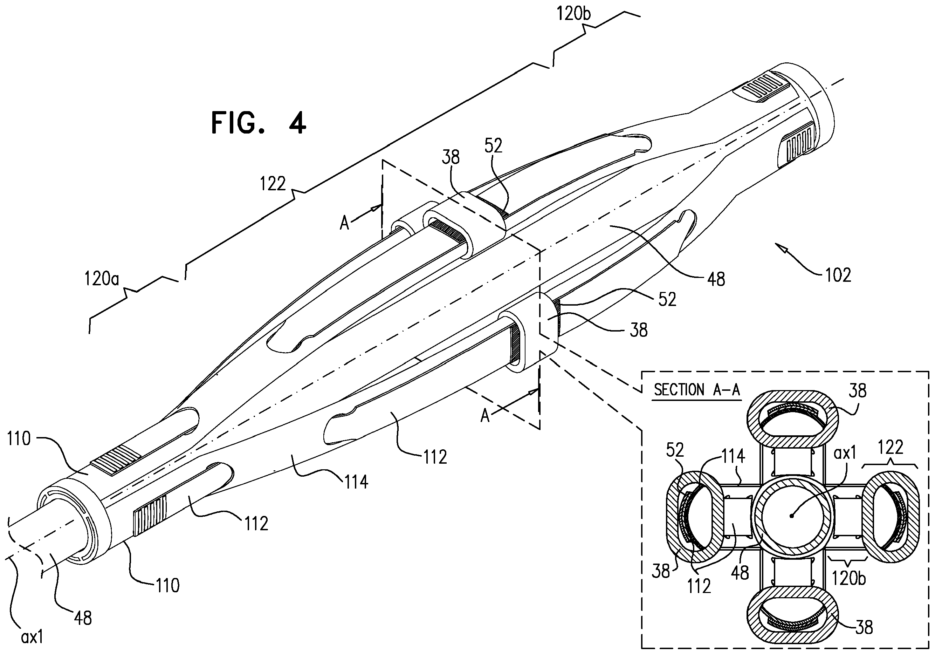

Reference is made to Fig. 4, which is a schematic illustration of an electrode device 102, in accordance with some applications of the invention. System 20 may comprise electrode device 102 instead of electrode device 22 or 62, *mutatis mutandis.* Electrode device 102 is typically identical to electrode device 22 or 62, described hereinabove, except where noted. Each subunit 110 of device 102 comprises a PCB 112 that is typically identical to PCB 32 or 72, *mutatis mutandis*; and a scaffold 114 that is typically identical to scaffold 34 of the *74, mutatis mutandis.* Scaffold 114 is shown without hinges, but may in fact have hinges such as hinges 66, described hereinabove.

As described hereinabove, the respective electrode regions of electrode devices 22 and 62 are generally straight and/or flat, such that in both the compressed and expanded states each electrode of a particular subunit is the same distance as the other electrodes of that subunit from the central longitudinal axis of the device. Each subunit 110 of device 102 has an electrode region 122 between two hinge regions 120a and 120b, and the electrode region has only one electrode 38, typically in the middle of the electrode region. Unlike electrode regions 42 and 82, electrode region 122 is convex (at least in its expanded state), with the single electrode 38 at the apogee of the electrode region. Because each electrode region only has a single electrode, having a straight and/or flat electrode region that is parallel with the longitudinal axis of the electrode device is less important for device 102 than it is for devices 22 and 62.

Fig. 4 also includes a cross-section of device 102, illustrating the juxtaposition of PCB 112 (including pad 52), electrode 38, scaffold 114, and control rod 48. This cross-section is typically also representative of electrode devices 22 and 62. The cross section illustrates, *inter alia,* that at the site of electrode 38 (e.g., throughout the electrode region), the PCB (and more specifically pad 52) is disposed radially outward from the scaffold (e.g., on a radially-outward-facing surface of the scaffold). The PCB (typically pad 52 thereof) is sandwiched between the scaffold and the portion of electrode 38 that faces radially outward (and that is the tissue-contact portion of the electrode).

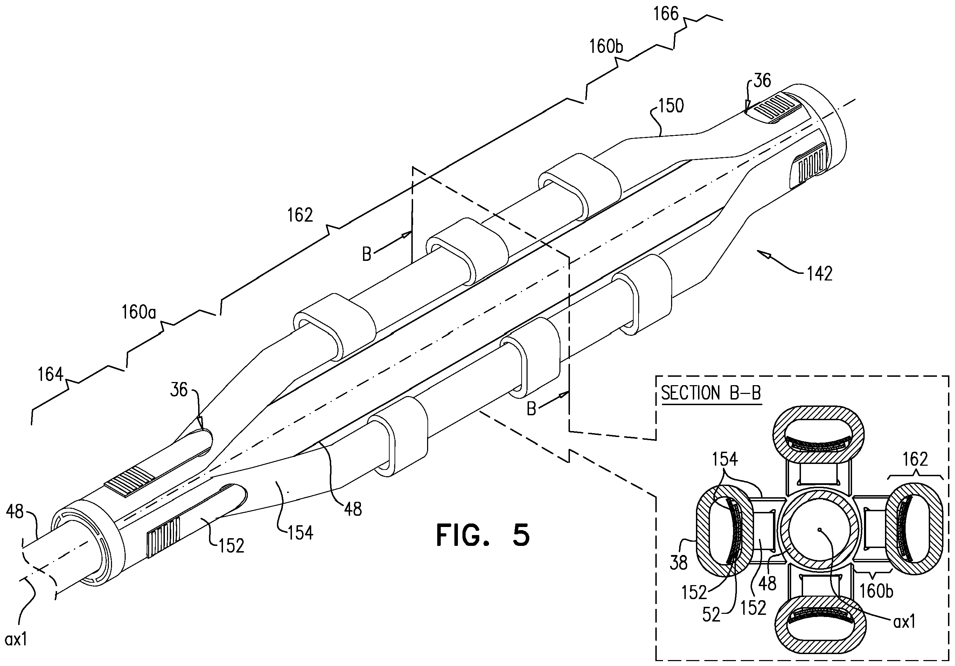

Reference is now made to Fig. 5, which is a schematic illustration of an electrode device 142, in accordance with some applications of the invention. System 20 may comprise electrode device 142 instead of electrode device 22, 62 or 102, *mutatis mutandis.* Electrode device 142 is typically identical to electrode device 22, described hereinabove, except where noted. Each subunit 150 of device 142 comprises a PCB 152 that is typically identical to PCBs described hereinabove except where noted; and a scaffold 154 that is typically identical to scaffolds described hereinabove except where noted. Scaffold 154 is shown without hinges, but may in fact have hinges such as hinges 66, described hereinabove.

In the electrode region of the electrode devices described hereinabove (and as shown in particular in the cross-section of Fig. 4), the PCB (and more specifically pad 52) is disposed radially outward from the scaffold (e.g., on a radially-outward-facing surface of the scaffold). In contrast, in the electrode region of electrode device 142, PCB 152 (and more specifically pad 52) is disposed radially inward from scaffold 154 (e.g., on a radially-inward-facing surface of the scaffold). The PCB (typically pad 52 thereof) is sandwiched between the scaffold and the portion of electrode 38 that faces radially inward (and that is not the tissue-contact portion of the electrode). Because electrode 38 is typically a ring, it can conduct current from pad 52 around PCB 152 and scaffold 154 to a portion of the electrode that faces radially outward (and that is the tissue-contact portion of the electrode).

Such a configuration of electrode device 142 allows its PCB to be disposed radially inward from its scaffold, at least in electrode region 162. Typically, in proximal and distal regions (164 and 166, respectively) of electrode device 142, PCB 152 is disposed radially outward from scaffold 154. Therefore this configuration of electrode device 142 facilitates the placement of a single passthrough site 36 at each hinge region (160a and 160b) of the electrode device.

The feature of the PCB being radially inward from the scaffold at the electrode region may be applied to the other electrode devices described herein, *mutatis mutandis.*

Reference is again made to Figs. 1A-5. For some applications, at least one of pads 52 is not connected to an electrode 38. For some such applications, that pad 52 serves as a return electrode for current that is applied via an electrode 38. For some such applications, PCB 32 has three pads 52, and only the middle pad 52 is connected to an electrode 38. For some such applications, the operator may choose which of the other pads will serve as the return electrode(s).

For some applications, the return electrode is disposed on control rod 48, and is not placed in contact with the blood vessel wall.

For some applications, rather than electrically connecting the PCB to wires 54 at a distal end of catheter 24, the PCB extends along the catheter, and thereby conductors 50 replace wires 54.

For some applications, when the electrode device (e.g., electrode device 22, 62, 102 or 142) is in its compressed state (i.e., is fully compressed), it has an outer diameter d1 of 1-2.5 mm. For some applications, when the electrode device is fully expanded, it has an outer diameter d2 of 4-15 mm (e.g., 4-8 mm). For some applications, when the electrode device is not subject to any radial force, it has an outer diameter that is larger than d2, and is typically smaller than d1 (e.g., 2-4 mm). For example, scaffold 34 may have shape memory that biases the electrode device to assume such a diameter. It is hypothesized that such a configuration reduces the force required to move the electrode device into its expanded state (and for some applications, into its compressed state).

Typically, the electrode device has a length of 15-25 mm.

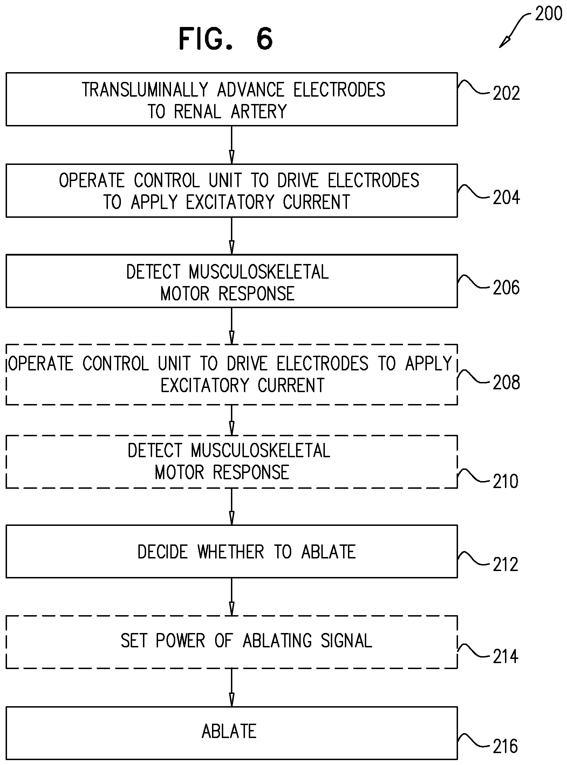

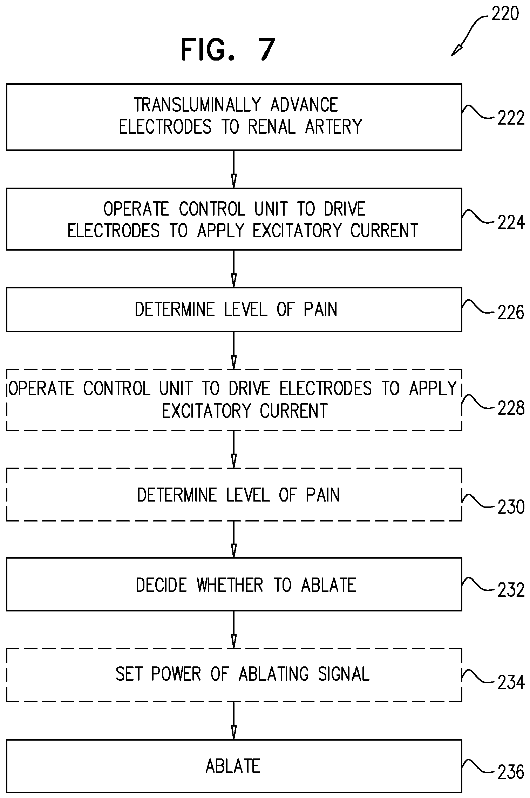

Reference is made to Figs. 6 and 7, which are flow charts of at least some steps in respective techniques 200 and 220 for use with a renal artery of a subject, in accordance with some applications of the invention. The technique of Fig. 6 relates to making decisions regarding renal nerve ablation based on detection of a musculoskeletal motor response. The technique of Fig. 7 relates to making such decisions based on pain detection. In both these techniques, high responsiveness typically indicates that ablation should not be performed at the particular site, or in the particular subject, or that a parameter of the ablation signal should be adjusted.

In the first step (202, 222) shown of each technique, electrodes are transluminally (typically transfemorally) advanced to the renal artery of the subject. For example, the electrodes may be electrodes 38 described hereinabove, and are advanced by advancing system 20 transfemorally. Subsequently, a control unit (e.g., control unit 240, described hereinbelow) is operated to drive the electrodes to apply excitatory current to adjacent tissue (204, 224). The excitatory current may have a frequency of greater than 5 Hz and/or less than 50 Hz (e.g., 5-50 Hz, e.g., 10-40 Hz, such as 20 Hz). The excitatory current may have an amplitude of greater than 1 mA and/or less than 200 mA (e.g., 1-200 mA, e.g., 1-100 mA, such as 50 mA). A response to the excitatory current is then detected (206, 226).

In technique 200, the response detected is a musculoskeletal motor response, such as movement of a limb (e.g., a leg, arm, or hand) or of a core muscle (e.g., a lower abdominal muscle) of the subject. That is, in certain circumstances, a musculoskeletal motor response may occur in response to the excitatory current being applied to the internal surface of the renal artery. This may occur, for example, due to the position of the electrodes within the renal artery, variations between subjects in the position of nerve tissue, and/or characteristics of the excitatory current (such as, but not limited to amplitude). The detection may be performed manually (e.g., by the operator observing the subject during application of the excitatory current), or automatically (e.g., using one or more accelerometers coupled to the subject).

In technique 220, the response detected is a pain response. That is, in certain circumstances, pain may be experienced in response to the excitatory current being applied to the internal surface of the renal artery. This may occur, for example, due to the position of the electrodes within the renal artery, variations between subjects in the position of nerve tissue, and/or characteristics of the excitatory current (such as, but not limited to amplitude). The detection is typically performed by the subject providing feedback, e.g., verbally or via a pain-level gauge.

For some applications of the invention, a second application of excitatory current may be applied (step 208, 228) and the detection of the motor or pain response is repeated (step 210, 230). The second excitatory current may have the same or different characteristics as the first application. For example, when the detected response is a motor response, the second excitatory current may have a frequency of greater than 1 Hz and/or less than 250 Hz (e.g., 1-250 Hz, e.g., 30-150 Hz, such as 90-110 Hz). (For some applications, the first excitatory current may also have this frequency.)

In response to the detection of the response, a decision is made whether to perform renal nerve ablation (i.e., to apply an ablating signal (step 212, 232)). The decision may be whether to apply the ablating signal (e.g., an ablating current, such as a Radio Frequency (RF) current) at the site at which the excitatory current was applied, or whether the subject him/herself is suitable for renal nerve ablation at all. If the motor or pain response is above a threshold magnitude (which may be effectively zero, or may be higher), the decision is typically to not ablate (at least not at the site at which the excitatory current was applied). For example, the motor or pain response being above the threshold magnitude may indicate that a motor or sensory nerve is close enough to also be ablated when ablation is performed.

For some applications, a decision not to ablate is made in response to both (i) detection of a motor or sensory response, and (ii) a small or absent increase in blood pressure. For some applications, a decision not to ablate is made even if a significant blood pressure increase occurs in combination with the motor or sensory response, e.g., taking into consideration the potential risk of ablating a motor or sensor nerve. For applications in which a decision is made not to ablate, the electrode device (and typically the entire system) is withdrawn from the subject without having applied ablative current to the renal artery since the introduction of the electrode device.

For some applications, prior to applying the ablating signal, the power of the ablating signal is set at least in part responsively to the detected response (step 214/234). For example, if a motor or pain response is detected, but is below a particular magnitude, the ablating signal may be set to an amplitude that is lower than if no motor or pain response were detected at all.

Step 216/236 is the application of the ablating signal. For some applications, the ablating signal is applied using the same electrode(s) via which the excitatory current was applied. For some applications, the ablating signal is applied via one or more separate, dedicated electrodes. For some applications, the ablating signal is a signal other than an electrical current, such as an ultrasound signal.

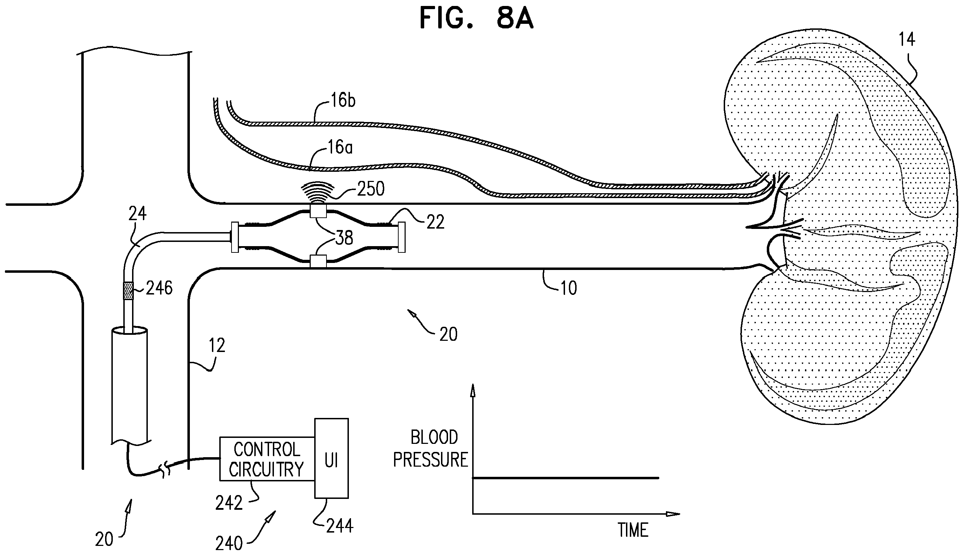

Reference is now made to Figs. 8A-H, which are schematic illustrations of a technique for performing renal nerve ablation, in accordance with some applications of the invention. In existing procedures, when performing ablation at multiple sites within a renal artery, the ablation device used is first advanced at the distal-most site required, and is progressively withdrawn proximally to each subsequent site. It is generally understood that this advantageously avoids the need to advance the device beyond the lesions made at previous ablation sites (and therefore also to withdraw the device back past the lesions); the lesions locally narrowing the artery and/or making the artery more susceptible to mechanical forces. As described with reference to Figs. 8A-H, the inventors propose that reversing the procedure advantageously facilitates sufficient but not excessive ablation of tissue of the renal artery for renal denervation.

Figs. 8A-H show a renal artery 10 of a subject branching from a descending aorta 12 of the subject, and leading to a kidney 14 of the subject. Two of the nerve fibers (16a and 16b) that innervate the kidney are shown. The paths shown for the two nerve fibers are intended to schematically illustrate that such fibers follow different paths (rather than to represent the actual paths in every individual). In particular, the paths shown are intended to illustrate that some fibers (e.g., fiber 16a) approach renal artery 10 closer to aorta 12 than do other fibers (e.g., fiber 16b).

As shown, system 20 (generally described hereinabove) typically further comprises a control unit 240 that comprises control circuitry 242 and a user interface (UI) 244. Control unit 240 (e.g., control circuitry 242 thereof) is configured to drive electrode device 22 (e.g., electrodes 38 thereof) to apply an excitatory current 250 and an ablating signal (e.g., ablating current) 260. In Figs. 8A-H, electrode device 22 is shown with a single electrode 38 per subunit, but as described hereinabove, the electrode device may comprise more electrodes per subunit. For some applications, control unit 240 drives the same electrode(s) to apply both the excitatory current and the ablating signal. Alternatively, separate electrodes are used. For some applications, system 20 comprises a physiological sensor (i.e., a sensor that detects a physiological parameter) such as a pressure sensor 246 for detecting blood pressure. As shown, pressure sensor 246 may be intracorporeal (e.g., disposed on catheter 24). However, pressure sensor 246 may alternatively be an extracorporeal pressure sensor (e.g., comprising a blood pressure cuff).

Catheter 24, with electrode device 22 comprising one or more electrodes 38, is advanced to a first portion of renal artery 10 (Fig. 8A). Electrode device 22 is driven by control unit 240 to apply excitatory current 250. Typically, control unit 240 (e.g., control circuitry 242 thereof) is configured such that the depth to which excitatory current 250 can effectively induce action potentials in nerve tissue is about the same as the depth to which ablating signal 260 can effectively ablate nerve tissue. Fig. 8A shows that at the first portion, neither of nerve fibers 16 is sufficiently close to artery 10 to be excited by the excitatory current (shown as the excitatory current not reaching either of the nerve fibers). Consequently, and as shown in the inset graph, the blood pressure of the subject does not change (or changes less than a threshold difference). That is, the physiological response to the excitatory current is less than a threshold response.

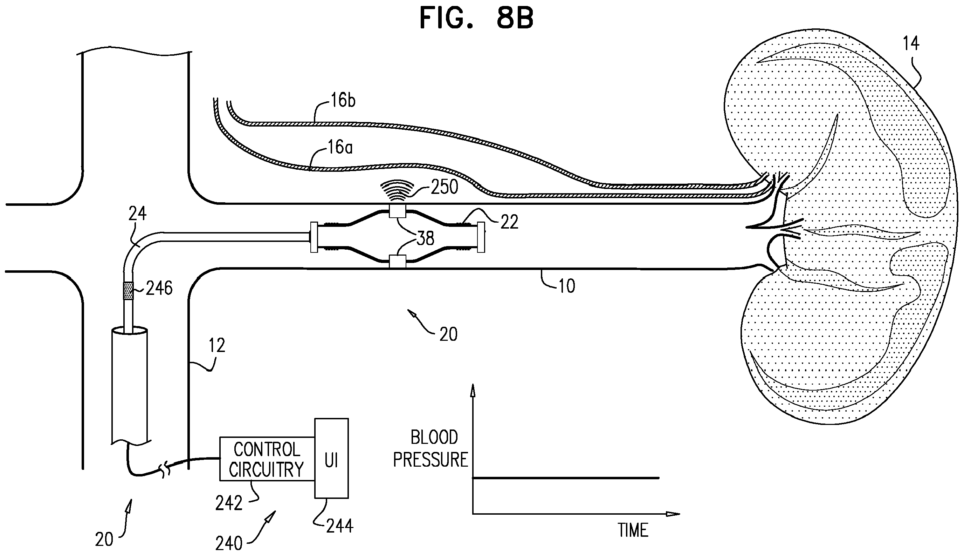

Catheter 24 is subsequently advanced further distally into renal artery 10, such that electrode device 22 is disposed at a second portion of the renal artery (Fig. 8B). This second portion may be described as "efferent" to the first portion because it is further along the nerve fibers from the central nervous system (CNS) than is the first portion. (Similarly, the second portion is further along the artery from the heart than is the first portion.) Again, the physiological response to the excitatory current is less than a threshold response because neither of nerve fibers 16 is sufficiently close to artery 10 to be excited by the excitatory current.

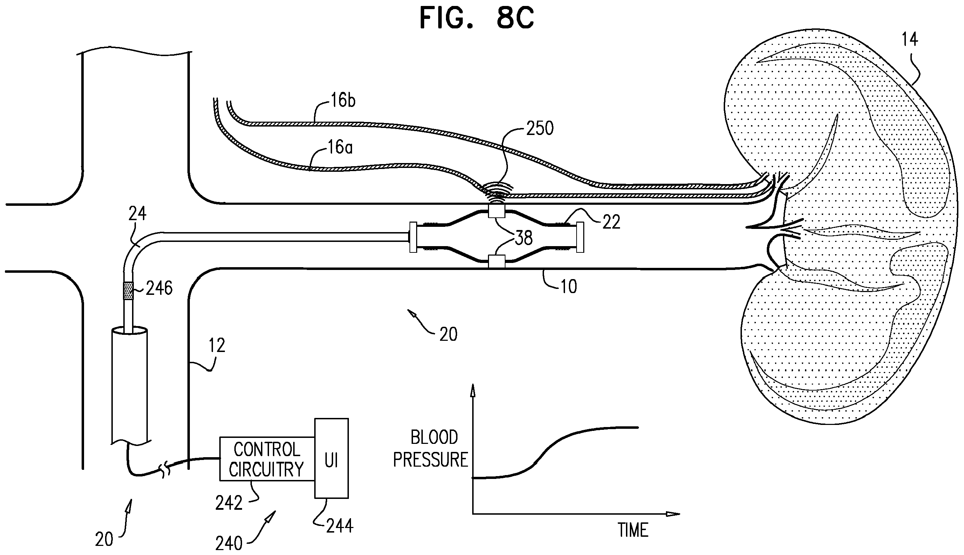

Once electrode device 22 is in the portion of renal artery 10 shown in Fig. 8C (for clarity, a "third portion" of the artery), fiber 16a (e.g., a "third portion" thereof), but not fiber 16b, is sufficiently close to artery 10 to be excited by excitatory current 250 (shown as the excitatory current reaching fiber 16a but not reaching fiber 16b), and therefore the physiological response to the excitatory current is greater than a threshold response (which may be the same or different to the threshold response used for other steps). As shown in the inset graph, the blood pressure of the subject increases in response to the induced action potentials. Both afferent and efferent action potentials have the potential to cause blood pressure to increase, and renal nerves comprise both afferent and efferent nerve fibers. However, the blood-pressure increase caused by afferent action potentials (e.g., a CNS response to the afferent action potentials) is typically faster than the blood-pressure increase caused by efferent action potentials (e.g., a hormonal response to the efferent action potentials).

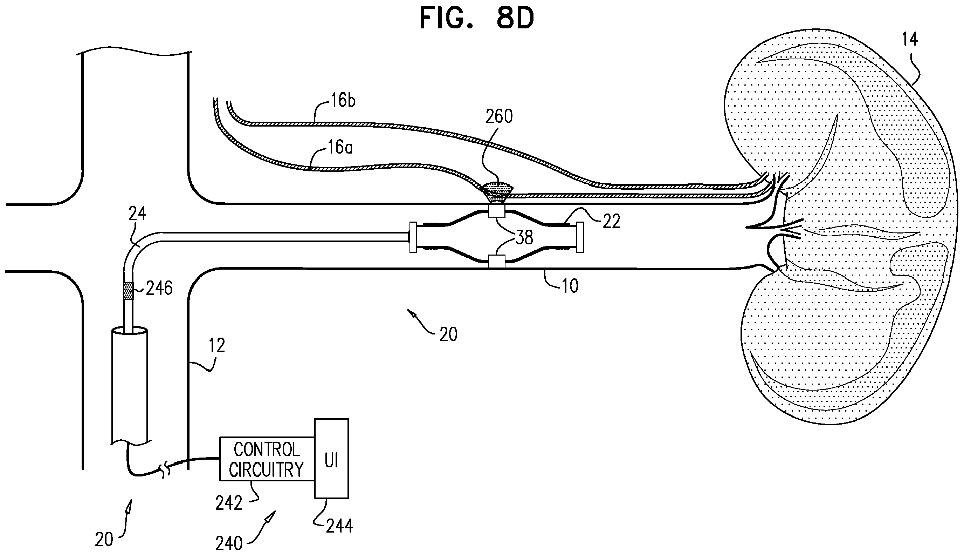

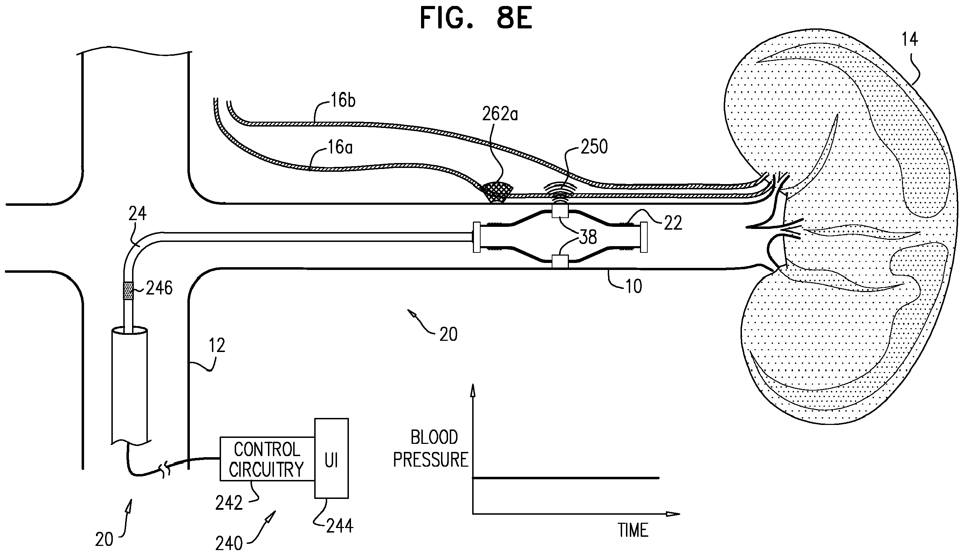

The increased blood pressure detected in the step of Fig. 8C indicates that fiber 16a (or a portion thereof) is sufficiently close. In response to this, ablating signal 260 is applied (Fig. 8D), which ablates fiber 16a at this site. It is to be noted that ablating signal 260 does not reach fiber 16b, and therefore fiber 16b is not ablated. The resulting lesion 262a is shown in Fig. 8E; it includes a portion of fiber 16a but none of fiber 16b.

Subsequently, catheter 24 is subsequently advanced further distally into renal artery 10, such that electrode device 22 is disposed at a fourth portion of the renal artery (Fig. 8E), and excitatory current 250 is again applied. Alternatively, the excitatory current may be applied without moving catheter 24 (i.e., the excitatory current may be applied to the same portion of the renal nerve to which the ablation energy was applied) (e.g., the fourth portion of the renal artery is the same as the third portion of the renal artery). Fiber 16b is insufficiently close to be excited by excitatory current 250, and although fiber 16a is sufficiently close, afferent action potentials induced therein by the excitatory current cannot propagate past lesion 262a. Therefore, the physiological response to the excitatory current is less than a threshold response (e.g., the same threshold or a different threshold that used in other steps). Fig. 8E shows an increase in blood pressure is not observed. (As described hereinabove, the response to efferent action potentials is slower and therefore not observed during the measurement window of the procedure.) It is to be noted that in a similar procedure in which electrode device 22 applies current at progressively proximal sites, excitatory current 250 would be applied on the proximal side of the previous lesion(s), and therefore afferent action potentials would be able to propagate toward the CNS and induce blood pressure increase. In response to this, an additional (and unnecessary) application of ablation signal would typically be applied even though the nerve fiber as a whole would already be incapable of carrying action potentials between the kidney and the CNS due to the previous lesion(s). The technique described with reference to Figs. 8A-H therefore reduces the likelihood of unnecessary ablation steps.

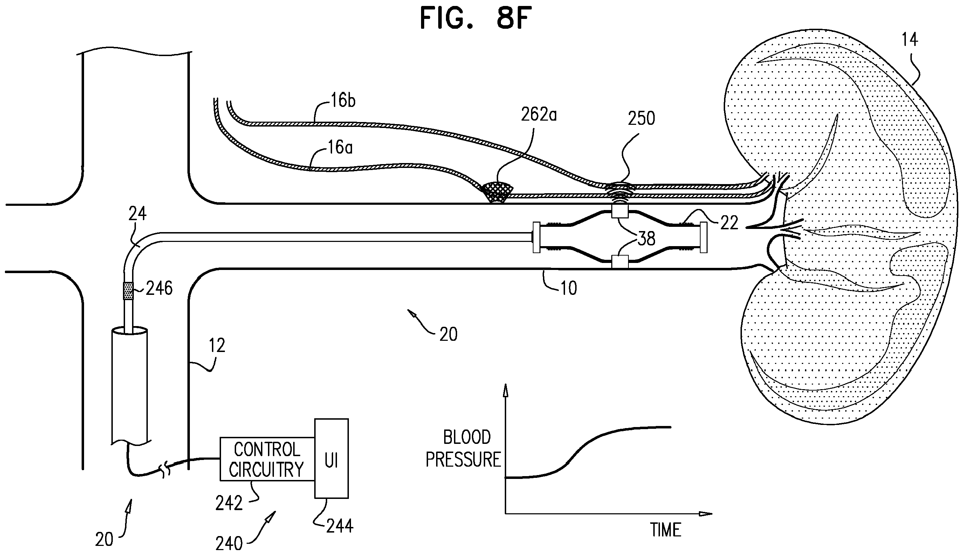

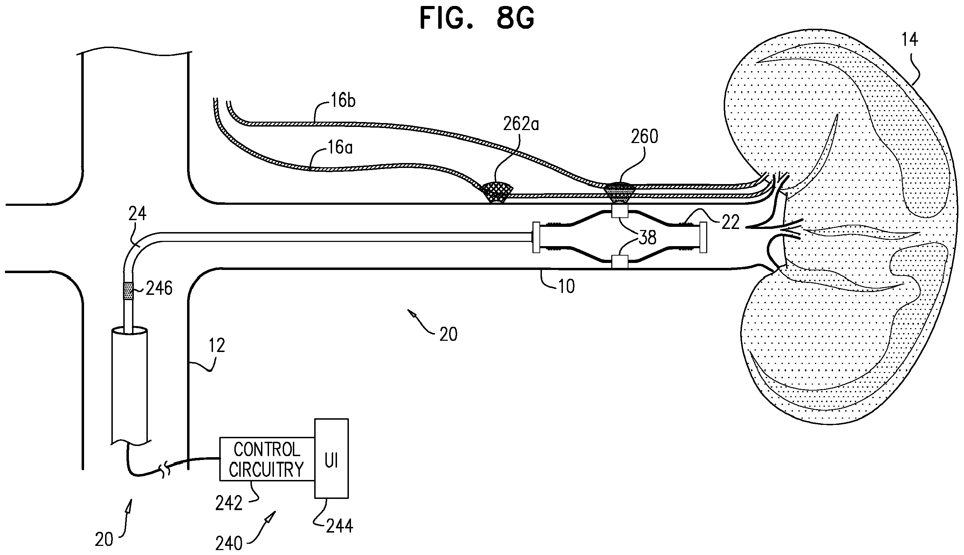

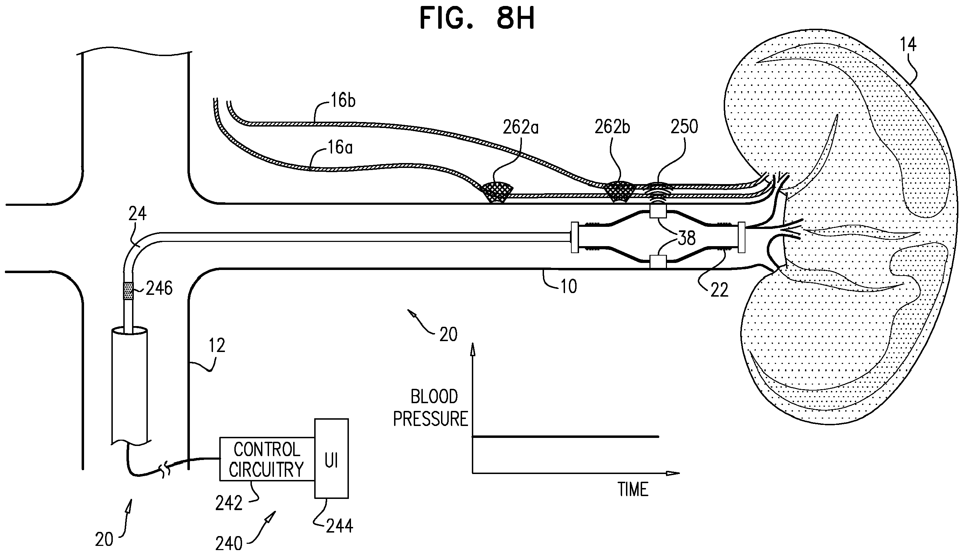

Subsequently, catheter 24 is advanced further distally into renal artery 10, such that electrode device 22 is disposed at a fifth portion of the renal artery (Fig. 8F), and excitatory current 250 is again applied. As indicated by the inset graph, the physiological response to the excitatory current is greater than a threshold response (e.g., the same threshold or a different threshold that used in other steps). Fig. 8F shows an increase in blood pressure is detected. Because lesion 262a prevents afferent action potentials from propagating along fiber 16a toward the CNS, the detection of this blood pressure increase indicates that a nerve fiber that was not ablated by a previous application of ablation signal 260 (in this case fiber 16b) has become sufficiently close to artery 10 for excitatory current 250 to induce action potentials therewithin. Therefore, in response to the detected blood pressure increase, another application of ablation signal 260 is subsequently applied at this site (Fig. 8G), thereby ablating fiber 16b (or the portion thereof that is sufficiently close). The resulting lesion 262b is shown in Fig. 8H.

Also shown in Fig. 8H is the subsequent application of excitatory current 250 at an even more distal site, and the lack of blood pressure increase in response thereto. As described with reference to Fig. 8E, *mutatis mutandis,* this indicates that, at that site, no nerve fiber exists that has not been ablated at some portion along its length.

Therefore, as described with reference to Figs. 8A-H, a method is provided, comprising:
(a) advancing a catheter to a first portion of a renal artery of the subject, the catheter having a plurality of electrodes coupled thereto;
(b) using one or more of the electrodes to apply ablating energy at a first portion of the renal artery (e.g., as described with reference to Fig. 8D);
(c) using one or more of the electrodes to apply excitatory current at a second portion of the renal artery that is at or efferent to the first portion (e.g., as described with reference to Fig. 8E);
(d) using a physiological sensor, sensing a physiological response of the subject to the excitatory current and determining whether the physiological response is below a threshold response (e.g., as also described with reference to Fig. 8E);
(e) subsequently, based upon determining that the physiological response is below the threshold response, advancing the catheter distally (e.g., as described with reference to Fig. 8F);
(f) subsequently, using one or more of the electrodes to apply excitatory current at a third portion of the renal artery that is the same as or efferent to the second portion (e.g., as also described with reference to Fig. 8F);
(g) using the physiological sensor, sensing a physiological response of the subject to the excitatory current applied at the third portion and determining whether the physiological response is above the threshold response (e.g., as also described with reference to Fig. 8F); and
(h) subsequently, based upon determining that the physiological response to the application of excitatory current at the third portion is above the threshold response, using one or more of the electrodes to apply ablating energy at the third portion (e.g., as described with reference to Fig. 8G).

For some applications, steps (d)-(h) are repeated at least twice.

For some applications, steps (d)-(h) are repeated at least until at least one of the electrodes of the catheter is disposed less than a distance of 1 cm (e.g., 0.5 cm) from the kidney connected to (i.e., supplied with blood by) renal artery 10. (It is to be noted that this includes the at least one electrode being disposed within the kidney.) For some such applications, subsequently to the catheter being within that distance from the kidney, the catheter is withdrawn from the subject without applying further ablating energy to the renal artery.

For some applications, and as described with reference to Fig. 8C, prior to step (b) above, excitatory current is applied at the first portion of the artery, and the ablation energy applied in step (b) is applied in response to the detected physiological response to the excitatory current being above a threshold response (e.g., the same or a different threshold to that of other steps).

For some applications, excitatory current is applied subsequent to one or more of the applications of ablation energy (e.g., at the same portion of the artery or efferent to the portion at which ablation energy was applied) in order to determine the success of the ablation step. If the response to the excitatory current is above a threshold response (e.g., the same or a different threshold to that of other steps), a further application of ablation energy may be applied (e.g., at the portion at which the previous application of ablation energy was applied, or at the portion at which the excitatory current was applied).

Typically, (i) prior to the application of excitatory current or ablation energy, the electrodes are placed in contact with artery 10 by expanding the electrode device, and (ii) when moving the electrodes between the portions of artery 10, the electrode device is compressed and then re-expanded.

For some applications of the invention, when the blood pressure of the subject does not change (or changes less than a threshold difference) in response to the excitatory current (e.g., as described with reference to Figs. 8A, 8B, and 8E), the amplitude of the excitatory current is increased and the excitatory current is reapplied (e.g., once or iteratively). It is hypothesized by the inventors that the increased amplitude increases the depth through the artery wall to which the excitatory current is effective at inducing action potentials in nerve tissue. If the increased amplitude results in the blood pressure increasing (or increasing more than the threshold difference), then rather than immediately advancing catheter 24 further distally into artery 10, ablation signal 260 may be reconfigured. For example, if the ablation signal is an RF current, a different (e.g., higher) amplitude may be used. Alternatively, the ablation modality to be used may be selected based on the amplitude of the excitatory current that successfully induced the blood pressure increase.

It is to be noted that, in Figs. 8A-H, the number of portions of the artery 10 to which current (excitatory or ablation) is applied is not intended to be limiting, but rather to illustrate the advantage of applying current at a progressively distal sites, rather than at progressively proximal sites.

It is to be noted that, despite the described advantages of the technique described with reference to Figs. 8A-H, the apparatus and techniques described elsewhere herein may alternatively or additionally be used in a distal-to-proximal manner.

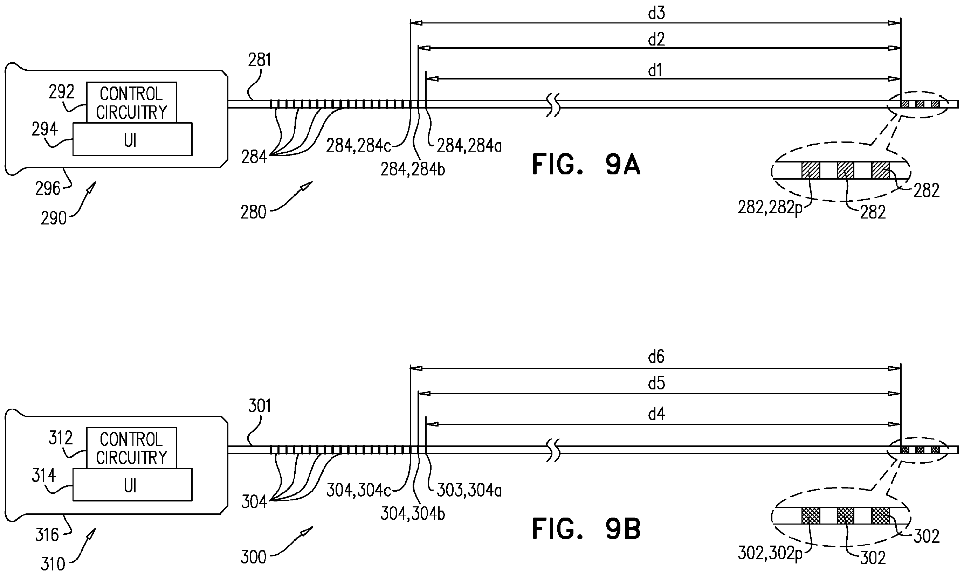

Reference is now made to Figs. 9A-B, which are schematic illustrations of a pair of catheter systems comprising a first catheter system 280 and a second catheter system 300, in accordance with some applications of the invention. For some applications it is desirable to separate identification/location of nerve fibers from ablation of the fibers. For example, it may be desirable to perform ablation at a different facility and/or at a later time (e.g., on a different day). The pair of catheters described with reference to Figs. 9A-B facilitate placement of an ablation electrode at a site that was previously identified as a target site for ablation (e.g., a site at which a relevant nerve fiber was located).

System 280 comprises a catheter 281 that has one or more stimulating electrodes 282 (typically at a distal portion of the catheter), and a plurality of longitudinally-distributed markers 284, all of which are located at least a distance d1 along the catheter from a proximalmost one of the stimulating electrodes, designated 282p. Typically, distance d1 is at least 20 cm and/or less than 2 m (e.g., 20-150 cm, such as 30-80 cm). System 300 comprises a catheter 301 that has one or more ablating electrodes 302 (typically at a distal portion of the catheter), and a plurality of longitudinally-distributed markers 304, all of which are located at least a distance d4 along the catheter from a proximalmost one of the ablating electrodes, designated 302p. Typically, distance d4 is at least 20 cm and/or less than 2 m (e.g., 20-150 cm, such as 30-80 cm).

Typically, (i) system 280 comprises a handle 296 to which catheter 281 is coupled, or is reversibly couplable, and (ii) system 280 comprises a handle 316 to which catheter 301 is coupled, or is reversibly couplable. Typically, the markers of each system are disposed on the catheter itself (i.e., on a flexible shaft thereof).

In Figs. 9A-B, a distalmost marker 284a of catheter 281 is distance d1 from proximalmost stimulating electrode 282p, and a distalmost marker 304a of catheter 301 is distance d4 from proximalmost ablating electrode 302p. Distance d1 and distance d4 are equal. Similarly, each of markers 284 is at a respective distance, along the catheter from electrode 282p, that is equal to a distance of a respective one of markers 304 from electrode 302p. Figs. 9A-B illustrate this by showing (i) a distance d2 along catheter 281 between a marker 284b and electrode 282p being equal to a distance d5 along catheter 301 between a marker 304b and electrode 302p; and (ii) a distance d3 along catheter 281 between a marker 284c and electrode 282p being equal to a distance d6 along catheter 301 between a marker 304c and electrode 302p.

Catheters 281 and 301 are each dimensioned such that, during normal use, at least one marker 284/304 of the catheter is disposed outside of the body of the subject. For example, for applications in which the electrode(s) of the catheter are transfemorally advanced into the renal artery, at least one of the markers is disposed outside of the body, close to the point of entry of the catheter into the body. The juxtaposition of the markers with respect to the point of entry (e.g., with respect to a trocar that provides access to the femoral artery) indicates the distance of the electrodes of the catheter from the point of entry.

When system 280 is used to identify/locate nerve fibers and target sites for ablation, the operator identifies, for each identified target site, the juxtaposition of markers 284 with respect to the point of entry. For example, the operator may record which of markers 284 is the most distal that is still visible (i.e., that has not yet entered the point of entry). When system 300 is subsequently used for ablation, the operator (who may be the same or a different operator) advances catheter 301 until the corresponding marker 304 is close to the point of entry but still visible. For any particular subject, having a particular vascular anatomy, the route to the renal artery is constant, and therefore electrodes 302 will be positioned at the site that was previously identified using electrodes 282.

As described for system 20, *mutatis mutandis,* system 280 and system 300 each typically further comprises a control unit that comprises control circuitry and a user interface (UI). System 280 comprises control unit 290 that comprises control circuitry 292 and a UI 294, and system 300 comprises control unit 310 that comprises control circuitry 312 and a UI 314.

Control unit 290 (e.g., control circuitry 292 thereof) is configured to drive electrodes 282 to apply an excitatory current. Control unit 310 (e.g., control circuitry 312 thereof) is configured to drive electrodes 302 to apply an ablation signal (e.g., an ablation current, such as an HF current). For some applications, separate control units 290 and 310 are not provided, but rather a single common control unit is provided, and is switchable (e.g., via the UI) between an excitatory mode in which the common control unit is configured to drive electrodes 282 to apply the excitatory current, and an ablation mode in which the common control unit is configured to drive electrodes 302 to apply the ablation signal. For such applications, catheters 281 and 301 are each reversibly electrically couplable to the common control unit. For some applications, the control unit is disposed within the handle of the system. For some such applications, a common handle is provided, and catheters 281 and 301 are each reversibly electrically couplable to the common handle.

For some applications, catheter 301 (or the entire of system 300) is provided without catheter 281 (or the entire of system 280), but rather is provided for use with catheter 281 (or system 280), whereby the distances between respective markers 284 and electrode 282p are predetermined (i.e., known), and markers 304 are distanced from electrode 302 accordingly. That is, for some applications, apparatus for use with a first catheter (catheter 301) is provided, the first catheter having a first plurality of longitudinally-distributed markers (markers 284) thereon, one or more ablating electrodes (electrodes 282) being coupled to the first catheter, all of the first plurality of markers being located at least 20 cm along the catheter from a proximalmost one of the ablating electrodes, the apparatus comprising: (a) a second catheter (catheter 301) having a second plurality of longitudinally-distributed markers (markers 304) thereon; and (b) one or more stimulating electrodes (electrodes 302) coupled to the second catheter, all of the second plurality of markers being disposed at least 20 cm from a proximalmost one of the stimulating electrodes, and each one of the second plurality of markers being at a respective distance from the proximalmost one of the stimulating electrodes that is equal to a distance of a respective marker of the first plurality of markers from the proximalmost one of the ablating electrodes.

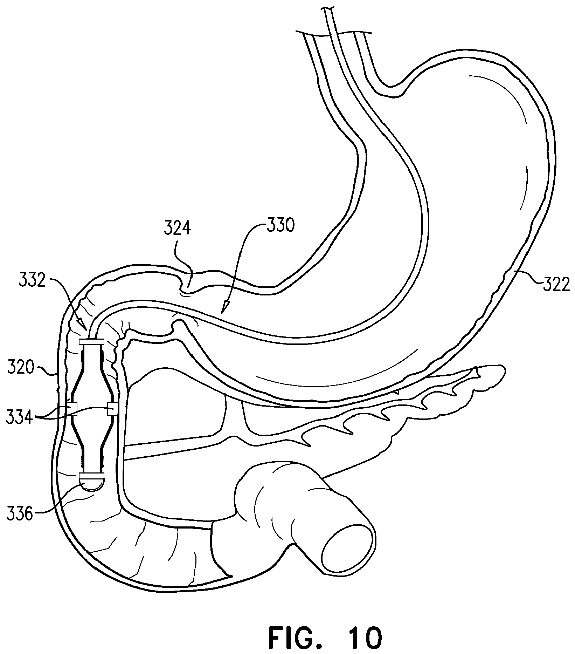

Reference is now made to Figs. 10-11, which are, respectively, a schematic illustration and a flow chart showing at least some steps of a technique for facilitating nerve ablation at a site other than the renal artery, in accordance with some applications of the invention. The example given in Figs. 10-11 is the facilitation of nerve ablation within the gastrointestinal tract of a subject.

A system 330, which may be similar to system 20 described hereinabove, *mutatis mutandis,* is advanced transesophageally such that an electrode device 332 at a distal end thereof moves through stomach 322 and pylorus 324, and into duodenum 320. For some applications, electrode device 332 has an expanded state in which it has an outer diameter of 20-40 mm (e.g., 25-35 mm).

One or more electrodes 334 of device 332 are used to apply excitatory current to (and through) the wall of the duodenum, and duodenal activity that is caused by the excitatory current is detected. Similarly to the use of blood pressure to identify the site of a renal nerve fiber, duodenal activity is used to identify the site of a nerve fiber that regulates duodenal activity. The identified nerve fiber (such as, but not limited to, a fiber of the vagus nerve), is then ablated using electrode device 332, e.g., by driving one or more electrodes 334 to apply an ablation signal (e.g., an ablation current, such as an RF current).

It is hypothesized that such ablation may reduce mixing within the duodenum, and thereby reduce calorie and/or glucose intake.

For some applications, the duodenal activity is identified using imaging (e.g., optical or ultrasound imaging). For example, system 330 (e.g., electrode device 332 thereof) may comprise an imaging device 336, such as a camera or ultrasound transceiver. For some applications, the duodenal activity is identified using electromyography (e.g., using the same or different electrodes disposed on device 332).

For some applications, prior to applying the excitatory current, one or more sensing electrodes (which may be electrodes 334, or may be other, dedicated, electrodes such as EMG electrodes) are used to detect duodenal activity. For example, electromyography may be used to identify a general area in which the nerve is likely disposed, and the stimulation-based technique described hereinabove is used to refine this location. For some applications, electromyography alone is used to locate the nerve fiber (i.e., without applying excitatory current). Alternatively or additionally, imaging (e.g., optical or ultrasound) may be used to facilitate identification of the general area.

That is, for some applications, a method for locating and ablating a nerve that regulates activity of a duodenum of a subject is provided, the method comprising: (a) advancing, into the duodenum of a subject, a distal portion of a catheter that comprises electrode device 332; (b) using the catheter to locate the nerve to a first degree of accuracy; and (c) subsequently, locating the nerve to a greater degree of accuracy, by (i) using the electrode device to apply an excitatory current to the duodenum, and (ii) using the catheter to detect a change in activity of the duodenum in response to the excitatory current. The locating to the first degree of accuracy, and/or the detecting of the change in activity of the duodenum may be performed using EMG electrodes and/or an imaging device on the catheter.

For some applications, after ablating the nerve, excitatory current is applied again, e.g., to determine how successful the ablation has been.

It is to be understood that the technique described with reference to Figs. 10-11 may be used, *mutatis mutandis,* with other parts of the gastrointestinal tract, or with non-gastrointestinal portions of the body, such as the superior mesenteric artery.

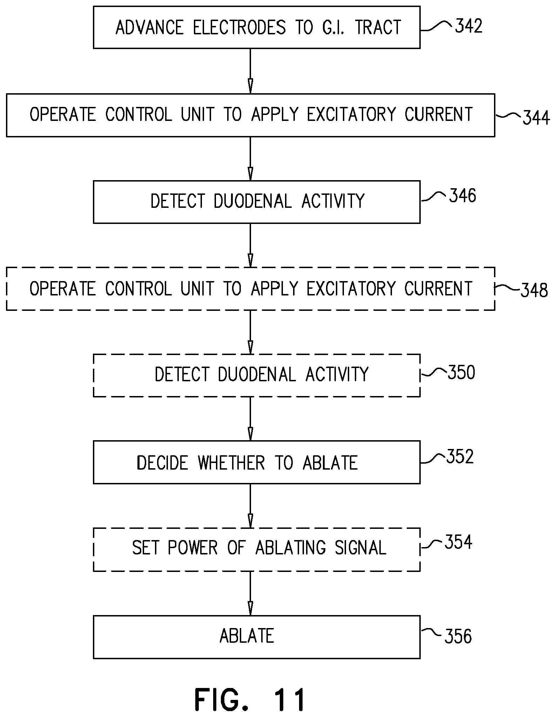

Fig. 11 is a flow chart of at least some of the steps of this technique. In step 342, electrodes are advanced to the gastrointestinal tract of the subject. Subsequently, a control unit (not shown in Fig. 10, but typically similar to other control units described herein) is operated to drive the electrodes to apply excitatory current to adjacent tissue (step 344). The excitatory current may have a frequency of greater than 1 Hz and/or less than 300 Hz (e.g., 1-300 Hz, such as 20-200 Hz). The excitatory current may have an amplitude of greater than 1 mA and/or less than 200 mA (e.g., 1-200 mA, such as 30-100 mA). A response to the excitatory current (i.e., duodenal activity) is then detected (step 346).

For some applications of the invention, a second application of excitatory current may be applied (step 348) and the detection of the duodenal activity is repeated (step 350). The second excitatory current may have the same or different characteristics as the first application.

In response to the detection of the response, a decision is made whether to perform nerve ablation, i.e., whether to apply an ablating signal (e.g., an ablating current, such as a Radio Frequency (RF) current) (step 352). The decision may be whether to apply the ablating signal at the site at which the excitatory current was applied, or whether the subject him/herself is suitable for such nerve ablation at all. If the duodenal response is below a threshold magnitude, the decision is typically to not ablate (at least not at the site at which the excitatory current was applied).

For some applications, prior to applying the ablating signal, the power of the ablating signal is set at least in part responsively to the detected response (step 354).

Step 356 is the application of the ablating signal. For some applications, the ablating signal is applied using the same electrode(s) via which the excitatory current was applied. For some applications, the ablating signal is applied via one or more separate, dedicated electrodes. For some applications, the ablating signal is a signal other than an electrical current, such as an ultrasound signal.

Reference is again made to Figs. 1A-11. For some applications, the excitatory and/or ablative current is applied in a monopolar manner (e.g., between a single electrode of the electrode device and an extracorporeal return electrode). For some applications, the current is applied in a bipolar manner (e.g., between two electrodes of the electrode device).

Although blood pressure is used as an example of a parameter detected in order to direct renal nerve ablation, for some applications of the invention one or more other parameters may be alternatively or additionally be detected and used, such as a parameter indicative of heart rate, heart rate variability, and/or blood flow of the subject.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. Apparatus for use with a first catheter having a first plurality of longitudinally-distributed markers thereon, one or more ablating electrodes being coupled to the first catheter, all of the first plurality of markers being located at least 20 cm along the catheter from a proximalmost one of the ablating electrodes, the apparatus comprising:
a second catheter having a second plurality of longitudinally-distributed markers thereon; and
one or more stimulating electrodes coupled to the second catheter,
all of the second plurality of markers being disposed at least 20 cm from a proximalmost one of the stimulating electrodes, and
each one of the second plurality of markers being at a respective distance from the proximalmost one of the stimulating electrodes that is equal to a distance of a respective marker of the first plurality of markers from the proximalmost one of the ablating electrodes.

2. The apparatus according to claim 1, further comprising the first catheter.

3. The apparatus according to claim 2, wherein the first catheter comprises an extracorporeal control unit that is electrically coupled to the one or more ablating electrodes, and is configured to drive the one or more ablating electrodes to apply a high-frequency ablation signal.

4. The apparatus according to claim 1, 2 or 3, wherein:
the first catheter comprises a first handle, and a first flexible and transluminally-advanceable shaft, and, the first plurality of markers are disposed on the first shaft, and
the first catheter comprises a second handle, and a second flexible and transluminally-advanceable shaft, and, the second plurality of markers are disposed on the second shaft.

5. The apparatus according to claim 4, further comprising the first catheter.

6. The apparatus according to any one of claims 1-5, wherein the second catheter comprises an extracorporeal control unit that is electrically coupled to the one or more stimulating electrodes, and is configured to drive the one or more stimulating electrodes to apply an excitatory current having a frequency of 5-50 Hz.
